**Europäisches Patentamt**

**European Patent Office**

**Office européen des brevets**

(11) Veröffentlichungsnummer: **0 276 377 B1**

# EUROPÄISCHE PATENTSCHRIFT

(45) Veröffentlichungstag der Patentschrift: **02.01.92**

(51) Int. Cl.⁵: **A61M 5/14**

(21) Anmeldenummer: **87115805.1**

(22) Anmeldetag: **28.10.87**

(54) **Druckinfusionsapparat.**

(30) Priorität: **29.01.87 DE 3702609**

(43) Veröffentlichungstag der Anmeldung:
**03.08.88 Patentblatt 88/31**

(45) Bekanntmachung des Hinweises auf die
Patenterteilung:
**02.01.92 Patentblatt 92/01**

(84) Benannte Vertragsstaaten:
**AT BE CH DE ES FR GB GR IT LI LU NL SE**

(56) Entgegenhaltungen:
**EP-A- 0 100 682**
**EP-A- 0 118 008**
**WO-A-82/03554**
**DE-A- 3 408 572**

(73) Patentinhaber: **B. Braun Melsungen AG**
**Carl-Braun Strasse**
**W-3508 Melsungen(DE)**

(72) Erfinder: **Heitmeier, Rolf, Dipl.-Ing.**
**Goethestrasse 8**
**W-3507 Baunatal(DE)**
Erfinder: **von der Haar, Friedrich ,**
**Prof.Dr.rer.nat.**
**Tilsiter Strasse 2**
**W-3508 Melsungen(DE)**

(74) Vertreter: **Selting, Günther, Dipl.-Ing. et al**
**Patentanwälte von Kreisler, Selting, Werner**
**Deichmannhaus am Hauptbahnhof**
**W-5000 Köln 1(DE)**

**Beschreibung**

Die Erfindung betrifft einen Druckinfusionsapparat gemäß der EP-A-0 100 682, die dem Oberbegriff der Patentansprüche 1 und 2 zugrundeliegt.

Die intravenöse Infusionstherapie verlangt Infusionsapparate, die eine hohe Genauigkeit, zeitliche Konstanz und Sicherheit des Volumenstroms realisieren. In Druckinfusionsapparaten werden Schlauchpumpen mit einem kalibrierten Schlauchsegment benutzt. Die Genauigkeit solcher Schlauchpumpen hängt wesentlich von der Maßgenauigkeit des Schlauchsegmentes ab, das einen Einmalartikel darstellt. Der Pumpenmotor ist ein Schrittmotor, der mit veränderbarer Ansteuerfrequenz betrieben wird. Nachteilig ist bei solchen Druckinfusionsapparaten, daß eine direkte Kontrolle des Volumenstromes nicht erfolgt.

Die EP-A-0 100 682 beschreibt einen Druckinfusionsapparat, bei dem die tatsächliche Infusionsrate gravimetrisch gemessen und zur Regelung der Pumpendrehzahl benutzt wird.

Der Volumenstrom wird durch die sich einstellende Gewichtsänderung des Flüssigkeitsbehälters ermittelt und zur Regelung der Schlauchpumpe benutzt. Auf diese Weise wird der Ist-Wert des Volumenstroms dem vorgegebenen Sollwert angeglichen. Eine Sicherheit gegen Fehlinformationen aus dem Meßwerterfassungskreis wird durch Überwachung der Pumpendrehzahl erzielt. Der gewünschten Infusionsrate wird über die vorgegebene Proportionalität zwischen Volumenstrom und Pumpendrehzahl ein plausibler Korrektur- oder Arbeitsbereich zugeordnet. Wird dieser Korrekturbereich überschritten, dann wird die Pumpe stillgesetzt und Alarm ausgelöst. Beim Betrieb eines Druckinfusionsapparates besteht ein Zielkonflikt der folgenden Art: Zur Ausregelung aller Störeinflüsse, wie Exemplarstreuungen des Einmalsystems, unterschiedliche Dichte, Veränderung und Dejustage der Pumpenmechanik, wird ein relativ großer Korrekturbereich benötigt; andererseits ist der Korrekturbereich zur Sicherheit gegen Fehldosierung möglichst klein auszulegen.

WO 82/3554 beschreibt einen Druckinfusionsapparat, bei dem zusätzlich zu der gravimetrischen Ermittlung des Volumenstroms durch Wiegen des Flüssigkeitsbehälters eine Tropfenzählung des Volumenstroms durchgeführt wird. Die Tropfenzählung soll eine schnellere Ermittlung des Volumenstroms ermöglichen. Es wird eine Soll-Tropfrate ermittelt, die durch den überlagerten Gewichtsregelkreis korrigiert wird. Die so ermittelte Tropfrate wird dem Tropfenregler als Ist-Wert zugeführt.

Der Erfindung liegt die Aufgabe zugrunde, einen Druckinfusionsapparat der im Oberbegriff der Patentansprüche 1 und 2 angegebenen Art zu schaffen, der eine hohe Sicherheit gegen Ausfälle einzelner Komponenten aufweist und dennoch einen großen Regelbereich hat und die eingestellte Infusionsrate mit hoher Genauigkeit einhält.

Eine erste Lösung dieser Aufgabe erfolgt erfindungsgemäß mit den im kennzeichnenden Teil des Patentanspruchs 1 angegebenen Merkmalen.

Bei dem erfindungsgemäßen Druckinfusionsgerät ist ein zusätzlicher Überwachungskanal vorgesehen, der unabhängig von dem Regelkreis ist und auf einem anderen physikalischen Meßprinzip beruht als der Regelkanal. Der Überwachungskanal führt eine selbständige Überwachung durch und spricht bei unzulässigen Abweichungen an, selbst wenn der Regelkanal solche Abweichungen nicht erkennt. Bei gravimetrischen Verfahren hat der Überwachungskanal eine besonders große Bedeutung, weil sich einfache Fehler in der Gewichtsmessung, wie z.B. Drift des Kraftaufnehmers, gefährlich für den Patienten auswirken. Dadurch, daß eine zweite Meßwerterfassung erfolgt, die auf einem anderen physikalischen Prinzip beruht als die zur Regelung benutzte Meßwerterfassung, werden Fehlfunktionen im Regelkreis mit hoher Sicherheit erkannt.

Eine zweite Lösung der Aufgabe ist im Patentanspruch 2 angegeben. Durch Vergleich der beiden durch unterschiedliche physikalische Meßprinzipien erhaltenen Meßwerte kann leicht erkannt werden, ob einer der Meßwerte falsch ist. Wenn dies erkannt wird, erfolgt Alarmauslösung. Auf diese Weise wird nicht nur der zur Regelung benutzte Meßwert überwacht, sondern auch der Meßwert des Überwachungskanals.

Zur Erhöhung der Überwachungsgenauigkeit können auch beide Lösungen gemeinsam angewandt werden.

Bei unterschiedlichen Tropfraten ergeben sich aus physikalischen Gründen unterschiedliche Tropfenvolumina. Das Tropfenvolumen hängt im wesentlichen ab von der Tropfenbildungsgeschwindigkeit, der Oberflächenspannung der Flüssigkeit und der Geometrie der Abtropfkante. Um eine hohe Genauigkeit der Überwachung zu ermöglichen, ist eine weitgehende Kompensation dieser Einflußgrößen notwendig. Mit dem Merkmal des Patentanspruchs 3 wird erreicht, daß die Umrechnung des Tropfens in ein äquivalentes Volumen in Abhängigkeit von der tatsächlichen Infusionsrate erfolgt. Bei hohen Infusionsraten ist das Tropfenvolumen im allgemeinen größer als bei geringen Infusionsraten.

Der erfindungsgemäße Druckinfusionsapparat kann auf einfache Weise mit einer Testschaltung versehen werden, die in regelmäßigen Abständen oder durch manuelle Auslösung einen Testzyklus mit einer simulierten falschen Tropfenzahl pro Zeiteinheit durchführt, um das Funktionieren des Alarmsystems zu überwachen.

Im folgenden wird unter Bezugnahme auf die Zeichnungen ein Ausführungsbeispiel der Erfindung näher erläutert.

Es zeigen:

Fig. 1 eine schematische Darstellung des Druckinfusionsapparates und

Fig. 2 Diagramme zur Erläuterung des Regelbereichs und der Alarmauslösung.

Der Druckinfusionsapparat weist eine Waage 10 auf, die aus einem Kraftsensor besteht, welcher an einer ortsfesten Basis 11 aufgehängt ist. An dem Haken 12 der Waage 10 hängt der Flüssigkeitsbehälter 13, der die Infusionsflüssigkeit enthält. Von dem Flüssigkeitsbehälter 13 führt ein Schlauch 14 über das Tropfsystem 15 zum Einlaß der volumetrischen Pumpe 16, die Vorzugsweise als Schlauchpumpe ausgebildet ist. Vom Auslaß der Pumpe 16 führt eine Schlauchleitung 17 zum Patienten.

Die Waage 10 mißt das Gewicht des Flüssigkeitsbehälters 13 und der an ihm hängenden Einrichtung. Die Gewichtsänderungen pro Zeiteinheit bilden ein Maß für den von der Pumpe 16 zum Patienten gepumpten Volumenstrom der Flüssigkeit. Das Ausgangssignal der Waage 10 wird dem ersten Meßwertumformer 18 zugeführt, der die von der Waage pro Zeiteinheit ermittelte Kraftdifferenz $\Delta F$ in ein Flüssigkeitsvolumen (in ml) umrechnet. Die hierzu erforderliche Dichteinformation der Infusionsflüssigkeit wird dem Meßwertumformer 18 über eine Leitung 19 zugeführt. Der Meßwertumformer 18 erzeugt den ersten Meßwert MW1 des Volumenstroms und gibt diesen ersten Meßwert als Ist-Wert an den Regler 20. Der Regler 20 empfängt ferner über Leitung 21 den eingestellten Sollwert SW des Volumenstroms. Aus der Differenz (MW1./. SW) bildet der Regler 20 ein Abweichungssignal, das für die Korrektur der Drehzahl der Pumpe 16 benutzt wird. Der Ausgang des Reglers 20 ist über einen elektronischen Schalter 22 und ggf. über einen (nicht dargestellten) Treiberverstärker mit dem Steuereingang der Pumpe 16 verbunden. Auf diese Weise wird ein Regelkreis gebildet, wobei aus der Gewichtsänderung des Flüssigkeitsbehälters 13 pro Zeiteinheit unter Berücksichtigung der Dichte der Infusionsflüssigkeit der tatsächliche Volumenstrom ermittelt wird und bei dem durch Regelung der Ist-Volumenstrom dem Sollwert SW angeglichen wird.

Das Tropfsystem 15 ist Bestandteil des Überwachungskreises. Er enthält den Tropfendetektor 23, der bei dem vorliegenden Ausführungsbeispiel als optischer Tropfendetektor, nämlich als Lichtschranke, ausgebildet ist und ein lichtemittierendes Element 24 sowie einen Fotoempfänger 25 enthält. Der Tropfensensor 23 erkennt jeden Tropfen, der in der Tropfkammer 26 herabfällt, welche im Verlauf des Schlauchsystems 14 angeordnet ist. Jeder Tropfen erzeugt einen Impuls, der dem zweiten Meßwertumformer 27 zugeführt wird. Der zweite Meßwertumformer 27 erhält außerdem von Leitungen 19 die Dichteinformation und von Leitung 21 den Sollwert SW. Aus der Tropfenzahl, der Dichteinformation und dem Sollwert SW berechnet der zweite Meßwertumformer 27 den zweiten Meßwert MW2 des Volumenstroms in ml. Dieser zweite Meßwert MW2 wird dem einen Eingang des Vergleichers 28 zugeführt, der an seinem anderen Eingang den Sollwert SW empfängt. Das Ausgangssignal des Vergleichers 28 steuert den elektronischen Schalter 22 in den nichtleitenden Zustand und erregt ferner eine Alarmvorrichtung 29, wenn der Betrag der Differenz (SW./.MW2) einen vorgegebenen Schwellenwert übersteigt. Durch das Sperren des Schalters 22 wird der Regelkreis unterbrochen und die Pumpe 16 wird stillgesetzt.

Beide Meßwerte MW1 und MW2 werden den Eingängen eines weiteren Vergleichers 30 zugeführt, der ein Ausgangssignal erzeugt, wenn der Betrag der Differenz (MW1 ./. MW2) einen vorgegebenen Schwellenwert übersteigt. Durch das Ausgangssignal des Vergleichers 30 wird ebenfalls der elektronische Schalter 22 in den nichtleitenden Zustand geschaltet und das Alarmgerät 29 aktiviert.

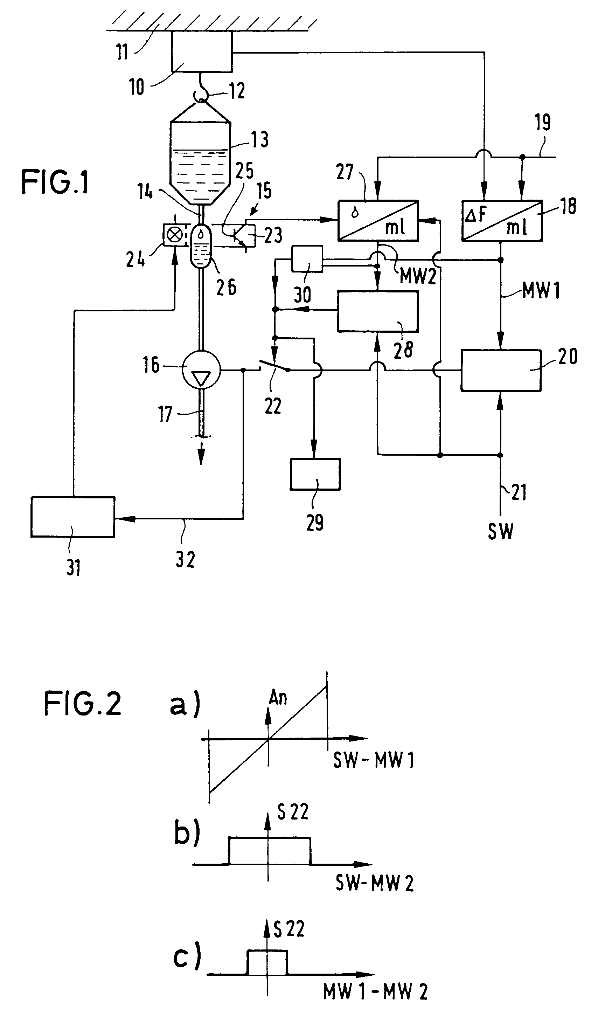

Figur 2a zeigt die Kennlinie des Reglers 20 und Figuren 2b und 2c zeigen die Kennlinie des Vergleichers 28 bzw. des Vergleichers 30. Gemäß Fig. 2a wird die Drehzahl n des Motors der Pumpe 16 linear zu dem Wert (SW ./. MW1) verändert. Diese Drehzahländerung $\Delta n$ ist innerhalb des Regelbereichs proportional zu der Differenz (SW ./. MW1). Wird der Regelbereich überschritten, dann wird Schalter 22 in den nichtleitenden Zustand geschaltet und Alarm ausgelöst.

Figur 2b zeigt den Schaltzustand S22 des Schalters 22 in Abhängigkeit von der Differenz (SW ./. MW2). Das hohe Impulsniveau stellt den Fall dar, daß der Schalter 22 leitend ist, also das Ausgangssignal des Reglers 20 zum Pumpenmotor überträgt, während das niedrige Niveau den Fall bezeichnet, daß Schalter 22 im nichtleitenden Zustand ist und Alarm erzeugt wird. Man erkennt, daß der Toleranzbereich gemäß Figur 2b kleiner ist als der Regelbereich gemäß Figur 2a.

Figur 2c zeigt die Kennlinien, nach der der Vergleicher 30 den Schalter 22 steuert. Der Toleranzbereich, den die Differenz (MW1 ./. MW2) einnehmen kann, ohne daß Alarm erzeugt wird, ist noch schmaler als der Toleranzbereich des Vergleichers 28 gemäß Fig. 2b.

Die Testschaltung 31 dient dazu, die Sicherheit gegen einen unbemerkten Ausfall des Überwachungskanals noch zu erhöhen. In regelmäßigen Abständen oder durch manuelle Auslösung erzeugt die Testschaltung 31 eine Reihe von Impulsen mit vorbestimmter Impulsfrequenz. Mit diesen Impulsen wird das lichtemittierende Element 24 des Tropfendetektors 23 dunkel getastet, so daß Tropfen simuliert werden. Auf diese Weise erkennt der Tropfendetektor eine falsche Tropfrate, die dazu führen muß, daß der Schalter 22 in den nichtleitenden Zustand geschaltet wird. Diese Reaktion wird über Leitung 32 der Testschaltung 31 mitgeteilt. Wenn Leitung 32 nach Simulation von Tropfenimpulsen die Abschaltung des Schalters 22 meldet, zeigt die Testschaltung 31 an, daß der Überwachungskreis funktioniert.

**Patentansprüche**

1. Druckinfusionsapparat mit
   einem Flüssigkeitsbehälter (13),
   einer die Flüssigkeit zum Patienten fördernden Pumpe (16),
   einer Waage (10), die Gewichtsänderungen des Flüssigkeitsbehälters (13) mißt,
   einem ersten Meßwertumformer (18), der aus den Gewichtsänderungen einen ersten Meßwert (MW1) für den Volumenstrom ermittelt,
   einer zwischen Flüssigkeitsbehälter (13) und Pumpe (16) angeordneten Tropfkammer (26),
   und einem Regler (20), der in Abhängigkeit von dem Volumenstrom den Pumpenantrieb derart regelt, daß der Volumenstrom sich einem vorgebbaren Sollwert (SW) annähert,
   **dadurch gekennzeichnet,** daß an der Tropfkammer (26) ein Tropfendetektor (23) vorgesehen ist, der an einen zweiten Meßwertumformer (27) zur Ermittlung eines zweiten Meßwertes (MW2) des Volumenstroms aus der pro Zeiteinheit ermittelten Tropfenzahl angeschlossen ist, daß nur einer der beiden Meßwerte (MW1) dem Regler (20) zugeführt ist und daß der andere Meßwert (MW2) einem Vergleicher (28) zugeführt ist, der die Pumpe (16) stillsetzt und Alarm auslöst, wenn dieser andere Meßwert (MW2) eine vorbestimmte Abweichung von dem Sollwert (SW) überschreitet.

2. Druckinfusionsapparat mit
   einem Flüssigkeitsbehälter (13),
   einer die Flüssigkeit zum Patienten fördernden Pumpe (16),
   einer Waage (10), die Gewichtsänderungen des Flüssigkeitsbehälters (13) mißt,
   einem ersten Meßwertumformer (18), der aus den Gewichtsänderungen einen ersten Meßwert (MWI) für den Volumenstrom ermittelt,
   einer zwischen Flüssigkeitsbehälter (13) und Pumpe (16) angeordneten Tropfkammer (26),
   und einem Regler (20), der in Abhängigkeit von dem Volumenstrom den Pumpenantrieb derart regelt, daß der Volumenstrom sich einem vorgebbaren Sollwert (SW) annähert,
   **dadurch gekennzeichnet,** daß an der Tropfkammer (26) ein Tropfendetektor (23) vorgesehen ist, der an einen zweiten Meßwertumformer (27) zur Ermittlung eines zweiten Meßwertes (MW2) des Volumenstroms aus der pro Zeiteinheit ermittelten Tropfenzahl angeschlossen ist, daß nur einer der beiden Meßwerte (MW1) dem Regler (20) zugeführt ist und daß ein Vergleicher (30) vorgesehen ist, der die Pumpe (16) stillsetzt und Alarm auslöst, wenn der erste und der zweite Meßwert (MW1,MW2) des Volumenstroms um mehr als einen vorgegebenen Betrag voneinander abweichen.

3. Druckinfusionsapparat nach Anspruch 1 oder 2,
   **dadurch gekennzeichnet,** daß der zweite Meßwertumformer (27) zur Berechnung des zweiten Meßwertes (MW2) aus der Tropfenzahl den Sollwert (SW) empfängt.

4. Druckinfusionsapparat nach einem der Ansprüche 1 bis 3,
   **dadurch gekennzeichnet,** daß eine Testschaltung (31) vorgesehen ist, die an dem Tropfsystem (15) eine Tropfenfrequenz simuliert und das Stillsetzen der Pumpe (16) als Reaktion auf diese Tropfenfrequenz überwacht.

5. Druckinfusionsapparat nach Anspruch 1,
   **dadurch gekennzeichnet,** daß der erste Meßwert (MW1) dem Regler (20) und der zweite Meßwert (MW2) dem Vergleicher (28) zugeführt ist.

**Claims**

1. Pressure infusion apparatus comprising

   a fluid container (13),

   a pump (16) for conveying the fluid to the patient, a weighing means (10) for measuring changes in the weight of the fluid container (13),

   a first transducer (18) for detecting a first measure value (MW1) for the volume flow from said changes in weight,

   a dripping chamber (26) arranged between the fluid container (13) and the pump (16), and

   a controller (20) for controlling the pump drive, in dependence of the volume flow, in such a manner that the volume flow approaches a desired value (SW) which can be predetermined,

**characterized in**

that the dripping chamber (26) has provided thereon a drop detector (23) connected to a second transducer (27) for detecting, from the detected drop number per time unit, a second measure value (MW2) of the volume flow, that only one (MW1) of the two measure values is supplied to the controller (20) and that the other measure value (MW2) is supplied to a comparator (28) which brings the pump (16) to a stop and triggers an alarm if said other measure value (MW2) exceeds a predetermined deviation from the desired value (SW).

2. Pressure infusion apparatus comprising

a fluid container (13),

a pump (16) for conveying the fluid to the patient,

a weighing means (10) for measuring changes in the weight of the fluid container (13),

a first transducer (18) for detecting a first measure value (MW1) for the volume flow from said changes in weight,

a dripping chamber (26) arranged between the fluid container (13) and the pump (16), and

a controller (20) for controlling the pump drive, in dependence of the volume flow, in such a manner that the volume flow approaches a desired value (SW) which can be predetermined,

**characterized in**

that the dripping chamber (26) has provided thereon a drop detector (23) connected to a second transducer (27) for detecting, from the detected drop number per time unit, a second measure value (MW2) of the volume flow, that only one (MW1) of the two measure values is supplied to the controller (20) and that a comparator (30) is provided which brings the pump (16) to a stop and triggers an alarm if the first and second measure values (MW1,MW2) of the volume flow deviate from each other by more than a predetermined value.

3. Pressure infusion apparatus according to claim 1 or 2,
**characterized in** that the second transducer (27) receives the desired value (SW) for calculating the second measure value (MW2) from the drop number.

4. Pressure infusion apparatus according to any one of claims 1 to 3,
**characterized in** that a test circuit (31) is provided for simulating a drop frequency in the dripping system (15) and for monitoring the stopping of the pump (16) in reaction to said drop frequency.

5. Pressure infusion apparatus according to claim 1,
**characterized in** that the first measure value (MW1) is supplied to the controller (20) and the second measure value (MW2) is supplied to the comparator (28).

**Revendications**

1. Appareil de perfusion sous pression, comportant
un réservoir de liquide (13),
une pompe (16) véhiculant le liquide jusqu'au patient, un dispositif de pesée (10) qui mesure des variations de poids du réservoir de liquide (13),
un premier convertisseur de valeur de mesure (18) qui détermine à partir des variations de poids, une première valeur de mesure (MW1) pour le débit volumique,
une chambre d'égouttement (26) disposée entre le réservoir de liquide (13) et la pompe (16),
et un dispositif de régulation (20) qui régule l'entraînement de pompe en fonction du débit volumique, de manière à ce que ce débit volumique se rapproche d'une valeur de consigne prédéfinie (SW),
caractérisé en ce que sur la chambre d'égouttement (26) est prévu un détecteur de goutte (23) qui est raccordé à un second convertisseur de valeur de mesure (27) destiné à élaborer une seconde valeur de mesure (MW2) du débit volumique à partir du nombre de gouttes par unité de temps qui a été déterminé, en ce que seule l'une des deux valeurs de mesure (MW1) est transmise au dispositif de régulation (20), et en ce que l'autre valeur de mesure (MW2) est transmise à un comparateur (28) qui arrête la pompe (16) et déclenche une alarme lorsque cette autre valeur de mesure (MW2) diffère de la valeur de consigne (SW) d'une valeur supérieure à un écart prédéfini.

2. Appareil de perfusion sous pression, comportant
un réservoir de liquide (13),
une pompe (16) véhiculant le liquide jusqu'au patient, un dispositif de pesée (10) qui mesure des variations de poids du réservoir de liquide (13),

un premier convertisseur de valeur de mesure (18) qui détermine, à partir des variations de poids, une première valeur de mesure (MW1) pour le débit volumique,

une chambre d'égouttement (26) disposée entre le réservoir de liquide (13) et la pompe (16),

et un dispositif de régulation (20) qui régule l'entraînement de pompe en fonction du débit volumique, de manière à ce que ce débit volumique se rapproche d'une valeur de consigne prédéfinie (SW),

caractérisé en ce que sur la chambre d'égouttement (26) est prévu un détecteur de goutte (23) qui est raccordé à un second convertisseur de valeur de mesure (27) destiné à élaborer une seconde valeur de mesure (MW2) du débit volumique à partir du nombre de gouttes par unité de temps qui a été déterminé, en ce que seule l'une des deux valeurs de mesure (MW1) est transmise au dispositif de régulation (20), et en ce qu'il est prévu un comparateur (30) qui arrête la pompe (16) et déclenche une alarme lorsque la première et la seconde valeur de mesure (MW1, MW2) du débit volumique diffèrent entre-elles d'une valeur supérieure à une valeur prédéfinie.

3. Appareil de perfusion sous pression selon la revendication 1 ou 2, caractérisé en ce que le second convertisseur de valeur de mesure (27) destiné au calcul de la seconde valeur de mesure (MW2) à partir du nombre de gouttes, reçoit la valeur de consigne (SW).

4. Appareil de perfusion sous pression selon l'une des revendications 1 à 3, caractérisé en ce qu'il est prévu un circuit de test (31) qui simule au système d'égouttement (15), une fréquence d'égouttement, et surveille la mise à l'arrêt de la pompe (16) en réaction à cette fréquence d'égouttement.

5. Appareil de perfusion sous pression selon la revendication 1, caractérisé en ce que la première valeur de mesure (MW1) est transmise au dispositif de régulation (20), et la seconde valeur de mesure (MW2) est transmise au comparateur (28).

FIG.1

FIG.2

a)

b)

c)